Europäisches Patentamt

European Patent Office

Office européen des brevets

(19)

(11) Veröffentlichungsnummer: **0 306 606**
**A1**

(12) # EUROPÄISCHE PATENTANMELDUNG

(21) Anmeldenummer: 88105926.5

(22) Anmeldetag: 10.05.85

(51) Int. Cl.⁴: **A61M 5/32**

(43) Veröffentlichungstag der Anmeldung:
**15.03.89 Patentblatt 89/11**

(60) Veröffentlichungsnummer der früheren
Anmeldung nach Art. 76 EPÜ: **0 201 611**

(84) Benannte Vertragsstaaten:
**AT BE CH DE FR GB IT LI LU NL SE**

(71) Anmelder: **B. Braun-SSC AG**
**Gerliswilstrasse 74**
**CH-6020 Emmenbrücke(CH)**

(72) Erfinder: **Bäumle,Hubert**
**Wanne**
**CH-6182 Escholzmatt(CH)**
Erfinder: **Schwöbel,Eckhard,Dipl.-Ing.**
**Reckenbühlstrasse 17**
**CH-6005 Luzern(CH)**

(74) Vertreter: **Maspoli, René A. et al**
**PATENTANWALTSBUREAU R.A. MASPOLI**
**Postfach 191**
**CH-8053 Zürich(CH)**

(54) **Einweg-Injektionsspritze mit zwei Kanülen.**

(57) Die neue Einweg-Injektionsspritze mit zwei Kanülen dient zur auswechselfreien Kanülenänderung zwischen Aspiration und Injektion.

Dazu sind die beiden Kanülen (01, 02) so angeordnet, dass bei der Verwendung immer zuerst die Aspirationskanüle (01) freiliegt, dass die Spitze der Injektionskanüle (02) gegenüber der Spitze der Aspirationskanüle (01) nach hinten versetzt vorliegt und dass die Halterung (05) der Aspirationskanüle und der dazugehörige Sitz um die Injektionskanüle herum konzentrisch-zylindrisch ausgebildet sind, um so die Spitze der Injektionskanüle bei der Herstellung und bei der Verwendung der Spritze zu schützen.

Fig. 2

EP 0 306 606 A1

## EINWEG-INJEKTIONSSPRITZE MIT 2 KANUELEN

Diese Erfindung betrifft eine Einweg-Injektionsspritze mit 2 Kanülen zum Aspirieren und Spritzen von Medikamenten in Form von Flüssigkeiten.

Injektionsspritzen mit abnehmbarer und daher auch auswechselbarer Kanüle sind bekannt, unter anderem aus den CH-Patenten 590 661 und 639 856 sowie aus der EP-Anmeldung, Ver. Nr. 0 047 442.

Spritzen für die Selbstinjizierung von Insulinlösungen werden unter anderem in der EP-Anmeldung, Ver. Nr. 0 045 367, beschrieben. Auf die bei derartigen Applikationen auftretenden Kontaminationsgefahren wird in dieser EP-Anmeldung zwar allgemein aufmerksam gemacht (Seite 3), dass es aber die Kanüle ist, die - vor allem bei mehrmaliger Benützung - die grösste Kontaminationswahrscheinlichkeit zeigt und somit die grösste Infektionsgefahr darstellt, wird nicht speziell erwähnt.

Auch das Arbeiten mit Wegwerf-Injektionsspritzen bringt dabei nur eine ungenügende Verringerung der Kontaminationsgefahr: Es ist die über längere Zeit benutzte Einstichstelle der Medikamentenflasche, die primär für die Uebertragung von Keimen auf die Kanüle in Frage kommt.

Ja selbst das Auswechseln der Kanüle zwischen dem Aspirieren und dem Injizieren kann noch zu Kontaminationen führen, vor allem wenn die Lösung in der Spritze während des Wechsels nicht abgedeckt bleibt oder wenn das Auswechseln die Berührung der Kanüle bedingt.

Eine doppelkanülige Entnahmevorrichtung für sterile Anwendungen ist in der EP-Anmeldung, Ver. Nr. 0 085 957, beschrieben und beansprucht. Speziell in den Vorrichtungen gemäss Figuren 5 und 9 dieser Anmeldung wird eine einstechbare Doppelkanüle dargestellt, bei denen die beiden Kanülen aneinanderliegend ausgeführt sind und die auch miteinander in den Lösungsbehälter eingestochen werden. Durch die eine der Kanülen wird Flüssigkeit entnommen, durch die andere wird - zwecks Druckausgleichs - keimfrei Luft in den Behälter eingeleitet.

Das Arbeiten mit einer Kanüle für Aspirieren bzw. Aufziehen und für Injizieren birgt zudem die Gefahr der Beschädigung der Kanüle, was sich beim Spritzen negativ auswirken kann.

Das DE Patent 378 629 lehrt und beansprucht eine Medizinalspritze, über deren Kanüle - nach der Einführung derselben in das Blutgefäss - ein Schutzrohr mit unscharfen Rändern und ohne Spitze, geschoben werden kann, um so das Innere des Blutgefässes vor der Kanülenspitze zu schützen. Das Schutzrohr kann, definitionsgemäss, nicht als Entnahme- bzw. Aspirierrohr für flüssige Medikamente verwendet werden.

Im Deutschen Gebrauchsmuster Nr. 1 678 482 wird ein Schutzröhrchen beschrieben, das beim Füllen der Spritze zwecks rein mechanischem Schutz der Einspritzkanüle auf dieselbe gesteckt wird. Das Schutzröhrchen ist für eine Mehrzahl von Spritzen gedacht, was dem wichtigsten Bestreben der Zwei-Kanülen-Spritze der Erfindung, der Kontaminationsminimalisierung nämlich, klar zuwiderläuft. Zudem kommt beim Anstechen des Medikamentenbehälters die Spitze der Injektionskanüle mit dem Verschluss in Berührung, was ebenfalls den erfindungsgemässen Intentionen zuwiderläuft. Die Spritze liegt zudem vor ihrer Benutzung unabgedeckt vor.

Ebenso ist die Anordnung der Spritzenbestandteile gemäss dem genannten DE-Gebrauchsmuster unbrauchbar für die Massenherstellung, die Lagerung und die Verwendung von Einweg-Injektionsspritzen:

- zuerst liegt immer schon die Injektionsspritze frei;

- die Spitze der Injektionskanüle liegt auf der gleichen Höhe wie diejenige der Aspirationskanüle und

- die Halterung bzw. der Sitz der von Hand aufgesetzten Aspirationskanüle ist konisch ausgebildet, was eine Beschädigung der Injektionsspritze beim Aufsetzen der Aspirationskanüle nicht ausschliesst.

Schliesslich lehrt auch das US-Patent 3 292 624 eine 2-Kanülen-Spritze. Die aussen angeordnete Kanüle ist diejenige zum Injizieren; die innere dient zum Aspirieren. Die Halterung bzw. der Sitz der Wechselkanüle ist konisch ausgebildet.

Erst durch die Einweg-Injektionsspritze mit 2 Kanülen gemäss der hier beschriebenen Erfindung aber wird grundsätzlich das Problem der auswechselfreien Kanülenänderung zwischen Aspiration und Injektion erkannt und gelöst.

Gemäss der Erfindung wird eine Einweg-Injektionsspritze mit zwei Kanülen zum Aspirieren und zum Spritzen von Medikamenten in Form von Flüssigkeiten gelehrt, aufweisend eine erste Kanüle 01 zur Aspirierung der Flüssigkeit in den Spritzenzylinder und zur eventuellen Durchmischung und/oder Entgasung der Flüssigkeit und eine zweite Kanüle 02 zur eigentlichen Injektion, wobei beide Kanülen geschützt vorliegen und konzentrisch um die SpritzenLängsachse angeordnet sind. Die Kanülen sind so angeordnet, dass zuerst die Aspirationskanüle zur Verwendung freiliegt, dass die Spitze der Injektionskanüle gegenüber der Spitze der Aspirationskanüle nach hinten versetzt vorliegt, in oder ausserhalb der Aspirationskanüle, und dass die Halterung der Aspirationskanüle und der dazugehörige Sitz um die Injektionskanüle herum konzentrisch-zylindrisch ausgebildet sind, so dass, bei der Einpassung der Aspirationskanüle mit Halterung während der Herstellung, bei der Handhabung während der Lagerung und beim Abnehmen der Aspirationskanüle mit Halterung während des Gebrauches die Spitze der Injektionskanüle geschützt ist.

Bei Anordnung der Injektionskanüle hinter der Aspirationskanüle ist zwischen den beiden ein Filter angeordnet, durch welches das aspirierte Flüssigkeitsmedium durchtreten muss; nach Abschluss der Aspiration wird das Filter, zusammen mit der Aspirationskanüle, entfernt.

Die beschriebenen Zwei-Kanülen-Spritzen werden aus den dafür geeigneten Materialien, insbesondere aus Kunststoffen für Spritzenkörper, Zylinder, Kolbenstange und Schutzklappe, Metall oder Kunststoff, speziell Stahl für die Kanüle und Synthese- oder Naturkautschuk für Kolben und mittels kostengünstiger Methoden hergestellt und sterilisiert.

Verwendung finden die erfindungsgemässen Zwei-Kanülen-Spritzen zur Aspirierung und Injizierung von Medikamenten in Form von Flüssigkeiten ohne Auswechseln der Kanüle; dazu werden Lagerungs- bzw. Transportphasen, Aspirationsphasen und Injektionsphasen zu unterscheiden sein.

Speziell werden die Einweg-Injektionsspritzen mit 2 Kanülen gemäss dieser Erfindung so verwendet, dass während der Lagerungs- bzw. Transportphase die erste Kanüle vor der zweiten Kanüle angeordnet ist, dass beide Kanülen von einer Schutzkappe geschützt sind, dass zur Aspirierung bzw. Mischung/Entgasung der Flüssigkeit die Schutzkappe entfernt wird und dass zur Injektion auch die erste Kanüle samt vollständiger Halterung entfernt wird.

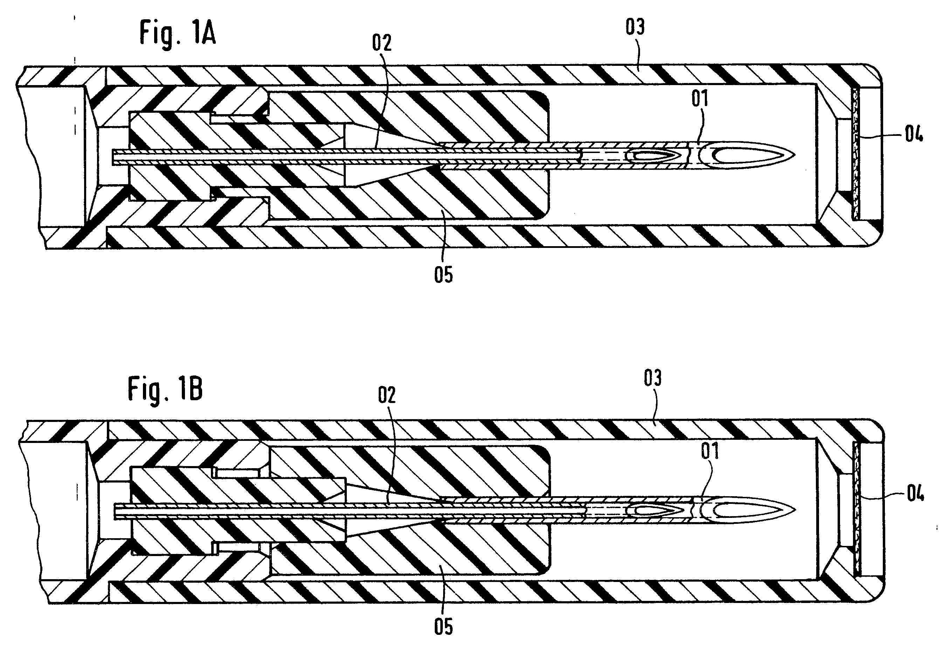

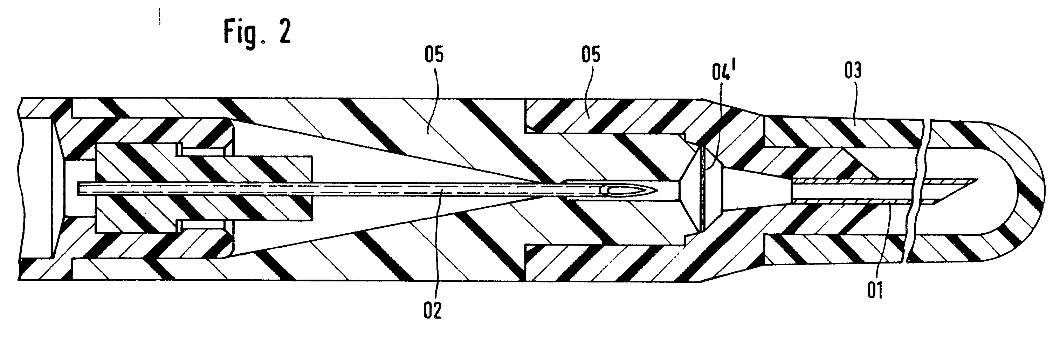

Im folgenden werden die erfindungsgemässen Spritzen anhand der beiliegenden Darstellungen in Figuren 1 und 2 genauer erläutert:

Die Figuren 1A und 1B zeigen eine erste Ausführungsform der erfindungsgemässen Einweg-Injektionsspritze mit 2 Kanülen in der Lagerungs- und Transportphase. Die zweite Kanüle 02 liegt dabei in der ersten 01; beide sind in der Kappe 03 mit Filter gegen aussen 04 versorgt. Zur Aspirierung wird die Schutzkappe entfernt. Nach dem Füllen des Spritzenzylinders mit der Flüssigkeit wird die erste Kanüle mit Halterung 05 entfernt. Dadurch erst wird die zweite Kanüle frei für die eigentliche Injektion.

Die Figur 2 zeigt eine zweite Ausführungsform der erfindungsgemässen Einweg-Injektionsspritze mit 2 Kanülen, ebenfalls in der Lagerungs- und Transportphase. Die erste Kanüle 02 liegt getrennt vor der zweiten Kanüle 02. Um die erste Kanüle liegt die Schutzkappe 03, die zwecks Aspiration entfernt wird. Beim Aufsaugen der Flüssigkeit passiert diese das Filter 04', welches aus einem dafür geeigneten Material hergestellt ist und in der Halterung 05 der ersten Kanüle befestigt ist. Nach der Aspiration wird die erste Kanüle mit ihrer Halterung und mit dem Zwischenfilter entfernt, Kanüle 02 ist für die Injektion frei.

Das oben angedeutete Filtrieren der Luft ist sehr wichtig bei der Aspiration des flüssigen Medikamentes aus einem starren Behälter; Die in den Behälter einströmende Luft muss keimfrei filtriert werden. Diese Keimfrei-Filtrierung der Luft kann mittels dem Fachmann leicht ersichtlicher Anpassung der Luftfilter und der zweiten Kanüle des erfindungsgemässen Spritzensystems geschehen.

Dass bei der erfindungsgemässen Spritze während der Lagerungs- und Aufziehphase die eigentliche Injektionskanüle vor Beschädigungen geschützt ist, ist hier ebenfalls anzumerken.

Die beschriebenen Gruppen der erfindungsgemässen Einweg-Injektionsspritze mit 2 Kanülen sind als beispielhafte Angaben anzusehen; ohne weiteres sind aufgrund des Erfindungskonzeptes weitere Ausführungsformen möglich.

## Ansprüche

1. Einweg-Injektionsspritze mit 2 Kanülen zum Aspirieren und zum Spritzen von Medikamenten in Form von Flüssigkeiten, aufweisend eine erste Kanüle (01) zur Aspirierung der Flüssigkeit in den Spritzenzylinder und zur eventuellen Durchmischung und/oder Entgasung der Flüssigkeit und eine zweite Kanüle (02) zur eigentlichen Injektion, wobei beide Kanülen geschützt vorliegen und konzentrisch um die Spritzen-Längsachse angeordnet sind,

dadurch gekennzeichnet,

- dass die Kanülen so angeordnet sind, dass zuerst die Aspirationskanüle zur Verwendung freiliegt,

- dass die Spitze der Injektionskanüle gegenüber der Spitze der Aspirationskanüle nach hinten versetzt vorliegt, in oder ausserhalb der Aspirationskanüle, und

- dass die Halterung der Aspirationskanüle und der dazugehörige Sitz um die Injektionskanüle konzentrisch-zylindrisch ausgebildet sind, so dass, bei der Einpassung der Aspirationskanüle mit Halterung während der Herstellung, bei der Handhabung während der Lagerung und beim Abnehmen der Aspirationskanüle mit Halterung während des Gebrauches, die Spitze der Injektionskanüle geschützt ist.

2. Einweg-Injektionsspritze mit 2 Kanülen gemäss Patentanspruch 1, weiter dadurch gekennzeichnet, dass bei Lage der Injektionskanüle hinter der Aspirationskanüle, zwischen den beiden Kanülen ein Filter angeordnet ist, durch welches das aspirierte Flüssigkeitsmedium durchtreten muss, welches Filter in die Halterung der Aspirationskanüle eingebaut ist, wobei nach der Aspiration die Aspirationskanüle und das Filter entfernt werden.

Fig. 1A

Fig. 1B

Fig. 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | US-A-4 059 112 (E.A. TISCHLINGER) <br> * Spalte 2, Zeilen 21-46; Figur 2 * <br> --- | 1 | A 61 M 5/32 |
| A | FR-A-2 366 026 (S. OIWA) <br> * Anspruch 1; Figur 4 * <br> --- | 1,2 | |
| D,A | DE-C- 378 629 (H. KOCH) <br> --- | | |
| D,A | DE-U-1 678 482 (L.H. ELLINGHAUSEN) <br> --- | | |
| D,A | US-A-3 292 624 (D. GABRIEL et al.) <br> ----- | | |

RECHERCHIERTE SACHGEBIETE (Int. Cl.4)

A 61 M 5/00

Der vorliegende Recherchenbericht wurde für alle Patentansprüche erstellt

| Recherchenort | Abschlußdatum der Recherche | Prüfer |
|---|---|---|
| BERLIN | 10-06-1988 | MASSALSKI W. |

EPO FORM 1503 03.82 (P0403)